(19) Europäisches Patentamt
European Patent Office
Office européen des brevets

(11) Veröffentlichungsnummer: **0 353 754 B1**

(12) **EUROPÄISCHE PATENTSCHRIFT**

(45) Veröffentlichungstag der Patentschrift: **02.12.92**

(51) Int. Cl.5: **A61K 31/55**, A61K 49/02, C07C 59/00

(21) Anmeldenummer: **89114359.6**

(22) Anmeldetag: **03.08.89**

Die Akte enthält technische Angaben, die nach dem Eingang der Anmeldung eingereicht wurden und die nicht in dieser Patentschrift enthalten sind.

# (54) Radioaktiv markierte Benzodiazepinderivate.

(30) Priorität: **05.08.88 US 229064**

(43) Veröffentlichungstag der Anmeldung:
**07.02.90 Patentblatt 90/06**

(45) Bekanntmachung des Hinweises auf die Patenterteilung:
**02.12.92 Patentblatt 92/49**

(84) Benannte Vertragsstaaten:
**AT BE CH DE ES FR GB IT LI LU NL SE**

(56) Entgegenhaltungen:
**EP-A- 0 027 214**
**EP-A- 0 300 341**
**EP-A- 0 313 291**
**US-A- 4 777 169**

**JOURNAL OF NUCLEAR MEDICINE. vol. 24, no. 5, Mai 1983, NEW YORK US Seiten 417 - 422; SCHOLL H. et al: "Bromine-75-labeled 1,4-benzodiazepines: potential agents for the mappingof benzodiazepine receptors in vivo:concise communication"**

**INTERNATIONAL JOURNAL OF RADIATION APPLICATIONS AND INSTRUMENTATION Part A vol. 39, no. 4, 1988, GB Seiten 353 - 356; ZECCA L. et al: "Synthesis and Biodistribution of an 123I labelled flunitrazepam derivative: a potential in vivo tracer for benzodiazepine receptors"**

(73) Patentinhaber: **F. HOFFMANN-LA ROCHE AG**
**Postfach 3255**
**CH-4002 Basel(CH)**

(72) Erfinder: **Carmann, Heinz, Dr.**
**Endresstrasse 92/4/3**
**A-1238 Wien(AT)**
Erfinder: **Hunkeler, Walter, Dr.**
**Im Stigler 32**
**CH-4312 Magden(CH)**

(74) Vertreter: **Lederer, Franz, Dr. et al**
**Lederer, Keller & Riederer Patentanwälte**
**Lucile-Grahn-Strasse 22**
**W-8000 München 80(DE)**

**EP 0 353 754 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft radiojodierte Benzodiazepinderivate der Formel

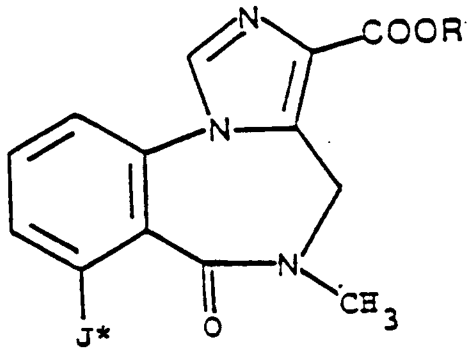

I

worin J* ein radioaktives Jod und R eine Alkylgruppe mit 1-4 C-Atomen bedeutet,
sowie deren Verwendung in der Diagnose von Krankheiten und Störungen des Hirns durch deren Fähigkeit das Hirn abzubilden.

Das radioaktive Jod ist vorzugsweise Jod 123. Die Alkylgruppe R ist vorzugsweise Methyl, Aethyl, Isopropyl, Sec.Butyl oder Tert.Butyl besonders bevorzugt Aethyl.

Die am meisten bevorzugte Verbindung gemäss der vorliegenden Erfindung ist Aethyl-7-$^{123}$Jod-5,6-dihydro-5-methyl-6-oxo-4H-imidazo[1,5-a][1,4]benzodiazepin-3-carboxylat.

Die Verbindungen der Formel I sind wertvoll zur Abbildung des Gehirns. Demgemäss zeigen die Verbindungen der Formel I eine rasche Akkumulierung im Gehirn, was darauf hinweist, dass sie die Fähigkeit aufweisen, die sogenannte Blut/Hirn-Barriere zu durchdringen. Die Verbindungen der Formel I zeigen eine rasche Lokalisierung der Radioaktivität im Hirn nach intravenöser Verabreichung.

Die Verbindungen der Formel I können zur Diagnose von Krankheiten oder Störungen des Hirns verwendet werden durch ihre Eigenschaft, Aenderungen in der Verteilung der Benzodiazepinrezeptoren im Hirn abzubilden. Auf diese Weise können die Verbindungen der Formel I zur Diagnose von Hirnkrankheiten oder Störungen, wie cerebrovasculare Erkrankungen (Z.B. Hirnschläge), neurologische Störungen (z.B. Epilepsie) und psychotische Krankheiten verwendet werden.

Die Verbindungen der Formel I können nach an sich bekannten Methoden hergestellt werden. So z.B. kann eine Verbindung der Formel I aus einer kalten Verbindung der Formel I, d.h. einer Verbindung, worin J ein stabiles Jod bedeutet, durch Austausch mit einem radioaktiven Jod, vorzugsweise Jod 123, hergestellt werden.

Für den Austausch von kaltem Jod durch heisses Jod kann Jod 123 in einer 0,1N Natriumhydroxid-Lösung verwendet werden. Diese Lösung wird mit einer Lösung der kalten Verbindung der Formel I während ungefähr einer Viertel bis ungefähr zwei Stunden erwärmt. Dieser Jod-Austausch wird in Gegenwart eines Lösungsmittels wie z.B. Eisessig durchgeführt.

Eine weitere und bevorzugte Methode zur Herstellung der Verbindungen der Formel I besteht darin, eine Verbindung der Formel II

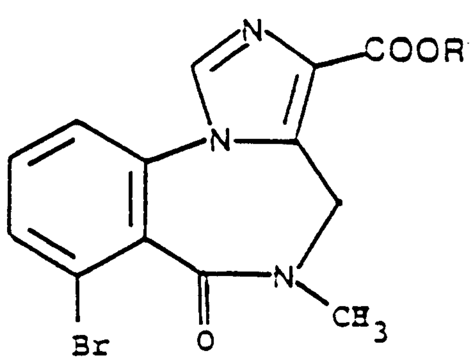

II

mit einem radioaktiven Jod umzusetzen.

Besonders bevorzugt wird Jod 123 verwendet.

Die gleichen Reaktionsbedingungen werden verwendet wie im Falle des Austausches des nicht-radioaktiven Jods durch Jod 123.

Die kalten Verbindungen der Formel I und die Verbindungen der Formel II sind entweder bekannt aus den Europäischen Patentpublikationen Nrn. 27214 oder 59389 oder aus der U.S. Patentschrift Nr. 4,316,839. Diese kalten Verbindungen können gemäss den Methoden, die in den obenerwähnten Europäischen Patentpublikationen sowie in der obenerwähnten U.S. Patentschrift beschrieben sind, hergestellt werden.

Wie obenerwähnt lokalisieren sich die radiojodierten Verbindungen gemäss vorliegender Verbindung nach intravenöser Verabreichung rasch im Hirn. In fast allen Fällen wird eine genügende Menge der verabreichten Dosis innerhalb von 2 bis 10 Min. im Hirn akkumuliert, was die Aufnahme von Scintiphotos erlaubt. Die Verbindungen gemäss vorliegender Erfindung zeigen während mindestens 60 Min. genügende Präsenz im Hirn, so dass signifikante Studien ausgeführt werden können.

Die radiojodinierten Verbindungen gemäss vorliegender Erfindung können in einem wässrigen oder wässrigen/alkoholischen Medium verabreicht werden. Solche Medien können auch konventionelle pharmazeutische Hilfsstoffe, wie z.B. pharmazeutisch annehmbare Salze zum Einstellen des osmotischen Druckes, Puffer, Konservierungsmittel und dergleichen enthalten.

Ein bevorzugter Träger für die paraenterale Verabreichung der Verbindungen der Formel I ist eine normale Kochsalzlösung, welche von ungefähr 0,5 Gewichtsprozent bis ungefähr 2 Gewichtsprozent eines geeigneten Konservierungsmittels enthält.

Die radioaktiven Benzodiazepine gemäss vorliegender Erfindung können einem Patienten für die diagnostische Abbildung des Hirns intravenös injiziert werden. Gemäss vorliegender Erfindung wird das radioaktive Benzodiazepin der Formel in einer injizierbaren Einheitsdosis verabreicht. Jede herkömmlichen Träger, wie sterile Kochsalzlösung, Plasma etc., können zur Herstellung der injizierbaren Lösung verwendet werden, die zur diagnostischen Abbildung gemäss vorliegender Erfindung verwendbar sind. Im allgemeinen enthält die zu verabreichende Einheitsdosis eine Radioaktivität von ungefähr 2 mCi bis ungefähr 10 mCi, vorzugsweise ungefähr 4 bis 5 mCi. Jede Menge an Verbindung gemäss vorliegender Erfindung, welche zur Abbildung des Hirns wirksam ist, kann jedoch gemäss vorliegender Erfindung injiziert werden. Die zu injizierende Lösung ist vorzugsweise eine Einheitsdosis von ungefähr 0,1 ml bis ungefähr 10 ml vorzugsweise von ungefähr 1 bis 5 ml und besonders bevorzugt von 4 bis 5 ml. Nach intravenöser Verabreichung wird das radioaktive Benzodiazepin der Formel I die Organe in vivo abbilden. Jede konventionelle Methode zur Visualisierung oder Abbildung für diagnostische Zwecke kann gemäss vorliegender Erfindung verwendet werden. In dieser Beziehung können scintigraphische Methoden zur Visualisierung oder Abbildung des Hirns verwendet werden.

Gemäss vorliegender Erfindung wird die Verbindung der Formel I und besondern Aethyl-7-$^{123}$Jod-5,6-dihydro-5-methyl-6-oxo-4H-imidazo[1,5-a][1,4]benzodiazepin-3-carboxylat in normaler Kochsalzlösung enthaltend 5% Glukose intravenös dem Menschen verabreicht. Die dem Menschen injizierte Dosis enthält 4 bis 5 mCi der radioaktiven Verbindung. Während 40 bis 60 Min. nach Injektion werden während 25 Min. Scintiphotos mit einer Gama Spect-Kamera aufgenommen. Gemäss vorliegender Erfindung wird vorzugsweise die Verbindung mit einer Dosis von 0,0543 mCi pro Kilogramm injiziert.

Die Verbindung der Formel I kann als freie Base oder als pharmazeutisch annehmbares Säureadditionssalz davon verabreicht werden.

Die nachfolgenden Beispiele illustrieren die Erfindung. Wenn nicht anderweitig vermerkt, sind alle Temperaturangaben in Grad Celsius angegeben.

Beispiel 1

Das Markierungsverfahren für den Halogenaustausch (Bromid-Iodid) wird in einem konischen Reaktionsgefäss, welches mit einem Teflon beschichteten Silikonseptum dicht geschlossen ist, durchgeführt. Jod 123-Aktivität bis zu 300 mCi) in 0,1n NaOH wird mit Hilfe eines schwachen Stroms von Stickstoff bei 90° zur Trockne eingedampft. Darauf wird 1 mg Aethyl-7-bromo-5,6-dihydro-5-methyl-6-oxo-4H-imidazo[1,5-a]-[1,4]benzodiazepin-3-carboxylat gelöst in 200 µl Eisessig zugegeben, worauf das Reaktionsgemisch während 1 Std. bei 150° erhitzt wird. Nach dem Abkühlen der Mischung löst man in 5 ml Wasser und reinigt durch HPLC. Die Reinigung erfolgte nach an sich bekannten Methoden. Markierung und Reinigung wurden in einem geeigneten Bleigefäss durchgeführt. Die Markierung war praktisch quantitativ.

Beispiel 2

Während der HPLC-Trennung wurde das Produkt des Hauptpeaks gesammelt und anschliessend zur Trockne eingedampft. Der Rückstand wurde in einer Lösung enthaltend 5% Glukose gelöst und zur Adsorption von während des Eindampfen freigesetzten Jods durch eine Silberpuder-Kolonne passiert. Nach Sterilfiltration und Einstellen der Aktivitätskonzentration auf 1 mCi/ml war das Aethyl-7-$^{123}$Jod-5,6-dihydro-5-methyl-6-oxo-4H-imidazo[1,5-a][1,4]benzodiazepin-3-carboxylat fertig zum Gebrauch. Die Produktqualität wurde mit Dünnschicht-Chromatography auf Silicagel überwacht, wobei mit Essigester/Ammoniumsulfat 200:1 entwickelt wurde.

Beispiel 3

Das in Beispiel 1 verwendete Ausgangsmaterial wurde wie folgt hergestellt:

Eine Mischung aus 167,95 g (624 mMol) 6-Brom-3,4-dihydro-4-methyl-2H-1,4-benzodiazepin-2,5(1H)-dion, 600 ml N,N-Dimethyl-p-toluidin und 800 ml Chloroform (über Aluminiumoxid filtriert) wird bei Siedetemperatur tropfenweise mit 160,80 g (1,05 Mol) Phosphoroxychlorid versetzt und während 4 Stunden unter Rückfluss gekocht. Man giesst die erhaltene Lösung auf ein vorgekühltes Gemisch aus 500 g Natriumbicarbonat und 2 l Wasser und rührt während 40 Minuten. Die anorganische Phase wird abgetrennt und dreimal mit Chloroform ausgeschüttelt. Die vereinigten Chloroformauszüge werden über Magnesiumsulfat getrocknet und das Chloroform eingedampft.

Inzwischen wird eine Lösung von 76 g (677 mMol) Kalium-t-butylat in 200 ml Dimethylformamid auf -45° abgekühlt, zuerst mit 71 g (625 mMol) Isocyanessigsäure-äthylester und anschliessen bei -50° bis -20° tropfenweise mit der obigen Lösung des Iminchlorids versetzt. Man entfernt die Kühlung, rührt das Gemisch während 1,5 Stunden, versetzt mit 13 ml Essigsäure, giesst auf 1800 ml Wasser und extrahiert fünfmal mit je 500 ml Methylenchlorid. Die vereinigten organischen Auszüge werden dreimal mit Wasser gewaschen, über Magnesiumsulfat getrocknet und eingedampft. Das Rohprodukt wird aus Methylenchlorid und Essigester unkristallisiert und liefert 156,80 g Aethyl 7-brom-5,6-dihydro-5-methyl-6-oxo-4H-imidazo-[1,5-a][1,4]benzodiazepin-3-carboxylat vom Schmelzpunkt 214-215° (69% der Theorie).

Beispiel 4

Jod-Jod-Austauschmarkierung wurde in der genau gleichen Weise durchgeführt wie der Brom-Jod-Austausch, wobei jedoch Aethyl-7-Jod-5,6-dihydro-5-methyl-6-oxo-4H-imidazo[1,5-a][1,4]benzodiazepin-3-carboxylat als Ausgangsmaterial verwendet wurde. Diese Markierung wurde durchgeführt um die Identität des markierten Produktes in HPLC und Dünnschicht-Chromatographie zu identifizieren.

Beispiel 5

Das Ausgangsmaterial, das in Beispiel 4 verwendet wird, wurde wie folgt hergestellt:

Eine Mischung aus 185,5 g (586,8 mMol) 3,4-Dihydro-6-jod-4-methyl-2H-1,4-benzodiazepin-2,5(1H)-dion, 670 ml N,N-Dimethyl-p-toluidin und 800 ml Chloroform (über Aluminiumoxid filtriert) wird bei Siedetemperatur tropfenweise mit 91,6 ml (979,9 mMol) Phosphoroxychlorid versetzt und während 2 Stunden unter Rückfluss gekocht. Man giesst die erhaltene Lösung auf ein vorgekühltes Gemisch aus 490 g Natriumcarbonat und 2 l Wasser und rührt während 40 Minuten. Die anorganische Phase wird abgetrennt und dreimal mit Methylenchlorid ausgeschüttelt. Die vereinigten Methylenchloridauszüge werden über Magnesiumsulfat getrocknet und das Methylenchlorid eingedampft. Inzwischen wird eine Lösung von 76,5 g (626,6 mMol) Kalium-t-butylat in 500 ml Dimethylformamid auf -50° abgekühlt, zuerst mit 65,2 ml (586,8 mMol) Isocyanessigsäure-äthylester und anschliessend bei -50° bis -15° tropfenweise mit der obigen Lösung des Iminchlorids versetzt. Man entfernt die Kühlung, rührt das Gemisch während 1 Stunde, versetzt mit 20 ml Essigsäure, giesst auf 1900 ml Wasser und extrahiert fünfmal mit Methylenchlorid. Die vereinigten organischen Auszüge werden dreimal mit Wasser gewaschen, über Magnesiumsulfat getrocknet und eingedampft. Nach Chromatographieren des Rückstandes an Kieselgel und Umkristallisieren aus Essigester erhält man 96,02 g Aethyl 5,6-dihydro-7-jod-5-methyl-6-oxo-4H-imidazo[1,5-a][1,4]-benzodiazepin-3-carboxylat vom Schmelzpunkt 244-246° (39,8% der Theorie).

Beispiel 6

Es werden je 3 Ratten (weiblich, Wistar, spf) zu sieben verschiedenen Zeitpunkten (2′, 10′, 20′, 40′, 1h, 6h, 15h) gemessen. Das Gewicht der Tiere lag zwischen 113 und 144 g. Die Fütterung erfolgte ad libitum. Die I.V. injizierten Dosen variierten von 184 bis 355 µCi in jeweils 0,2 ml.

| Prozent der injizierten Aktivität pro Gramm des entsprechenden Organs (bei der Ratte) | | | | | | | |
|---|---|---|---|---|---|---|---|
| | 2' | 10' | 20' | 40' | 60' | 6h | 15h |
| Blut | 0,70 | 0,87 | 0,46 | 0,17 | 0,11 | 0,04 | 0,013 |
| Hirn | 2,12 | 3,22 | 2,99 | 2,70 | 1,80 | 0,05 | 0,001 |
| Schilddrüse | 0,60 | 0,54 | 1,22 | 0,08 | 0,06 | 0,03 | 0,007 |
| Leber | 2,38 | 5,66 | 2,62 | 0,58 | 0,19 | 0,02 | 0,016 |
| Milz | 0,60 | 0,53 | 0,29 | 0,14 | 0,07 | 0,02 | 0,010 |
| Nieren | 2,68 | 7,06 | 5,26 | 1,35 | 0,68 | 0,03 | 0,010 |
| Magen | 0,19 | 0,41 | 0,35 | 0,27 | 0,48 | 0,73 | 0,080 |
| Darm | 0,75 | 1,10 | 1,73 | 2,02 | 1,97 | 1,55 | 0,143 |
| Lunge | 1,22 | 0,87 | 0,44 | 0,15 | 0,10 | 0,03 | 0,011 |
| Herz | 0,86 | 0,66 | 0,30 | 0,10 | 0,06 | 0,02 | 0,007 |
| Ovar | 0,88 | 0,73 | 0,53 | 0,22 | 0,16 | 0,03 | 0,011 |
| Femur | 0,54 | 0,45 | 0,24 | 0,10 | 0,07 | 0,02 | 0,007 |
| Blase | 0,59 | 1,07 | 0,69 | 0,24 | 0,42 | 0,57 | 0,023 |
| Muskel | 0,68 | 0,42 | 0,20 | 0,09 | 0,04 | 0,01 | 0,003 |

| Prozent der injizierten Aktivität in den entsprechend Organen (bei der Ratte) | | | | | | | |
|---|---|---|---|---|---|---|---|
| | 2' | 10' | 20' | 40' | 60' | 6h | 15h |
| Hirn | 3,11 | 5,38 | 4,20 | 4,02 | 2,42 | 0,08 | 0,0015 |
| Schilddrüse | 0,18 | 0,15 | 0,18 | 0,03 | 0,02 | 0,008 | 0,0015 |
| Leber | 16,90 | 34,37 | 17,61 | 3,90 | 1,29 | 0,15 | 0,107 |
| Milz | 0,26 | 0,21 | 0,11 | 0,06 | 0,03 | 0,008 | 0,0035 |
| Nieren | 3,15 | 7,48 | 6,25 | 1,53 | 0,79 | 0,04 | 0,009 |
| Magen | 0,81 | 1,20 | 1,28 | 1,37 | 1,96 | 1,35 | 0,346 |
| Darm | 9,28 | 12,70 | 22,53 | 25,54 | 28,48 | 19,50 | 1,012 |
| Lunge | 1,04 | 0,81 | 0,40 | 0,15 | 0,09 | 0,03 | 0,0095 |
| Herz | 0,58 | 0,37 | 0,17 | 0,06 | 0,03 | 0,009 | 0,0035 |
| Ovar | 0,09 | 0,06 | 0,05 | 0,02 | 0,02 | 0,003 | 0,0008 |
| Femur | 0,39 | 0,25 | 0,14 | 0,06 | 0,04 | 0,01 | 0,004 |
| Blase | 0,03 | 0,06 | 0,03 | 0,01 | 0,03 | 0,09 | 0,0022 |
| Restkörper | 63,94 | 29,92 | 25,92 | 11,04 | 7,09 | 2,88 | 1,30 |
| Totalkörper | 99,75 | 92,96 | 78,87 | 47,80 | 43,17 | 24,16 | 2,80 |
| Kot + Urin | 0,05 | 6,17 | 19,39 | 48,72 | 51,71 | 48,81 | 42,69 |

## Patentansprüche

## Patentansprüche für folgende Vertragsstaaten : AT, BE, CH, DE, FR, GB, IT, LI, LU, NL, SE

**1.** Ein radiojodiertes Benzodiazepin-Derivat der Formel

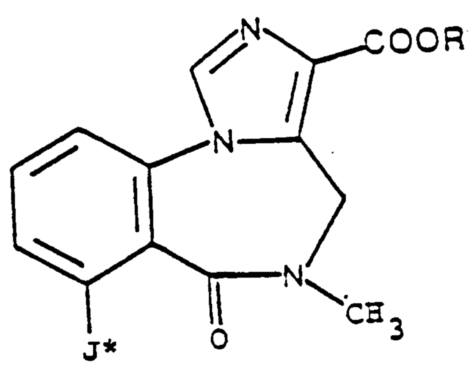

I

worin R niederes Alkyl mit 1-4 C-Atomen und J* ein radioaktives Jod darstellt.

**2.** Verbindung gemäss Anspruch 1, dadurch gekennzeichnet, dass das radioaktive Jod Jod 123 ist.

**3.** Aethyl-7-$^{123}$jod-5,6-dihydro-5-methyl-6-oxo-4H-imidazo[1,5-a][1,4]benzodiazepin-3-carboxylat.

**4.** Verfahren zur Herstellung einer Verbindung der Formel I von Anspruch 1, dadurch gekennzeichnet, dass man entweder

a) eine Verbindung entsprechend Formel I jedoch mit einem stabilen nicht-radioaktiven Isotop von Jod mit einem radioaktiven Jod markiert oder

b) eine Verbindung der Formel

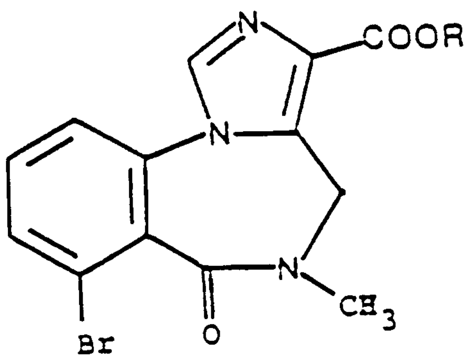

II

mit einem radioaktiven Jod umsetzt.

**5.** Verfahren nach Anspruch 4, dadurch gekennzeichnet, dass Jod 123 als radioaktives Isotop von Jod verwendet wird.

**6.** Zusammensetzung zur Abbildung des Hirns enthaltend eine Verbindung der Formel

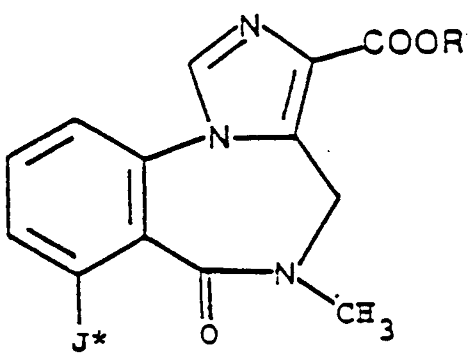

I

worin J* ein radioaktives Jod und R eine Alkylgruppe mit 1-4 C-Atomen bedeutet, und einen geeigneten Träger für Injektionszwecke.

**7.** Zusammensetzung gemäss Anspruch 6, dadurch gekennzeichnet, dass die Verbindung Aethyl-7-$^{123}$-jod-5,6-dihydro-5-methyl-6-oxo-imidazo[1,5-a][1,4]benzodiazepin-3-carboxylat ist.

**8.** Zusammensetzung gemäss Anspruch 6, enthaltend ungefähr 2 bis ungefähr 10 mCi der Verbindung der Formel I.

**Patentansprüche für folgenden Vertragsstaat : ES**

**1.** Verfahren zur Herstellung einer Verbindung der Formel

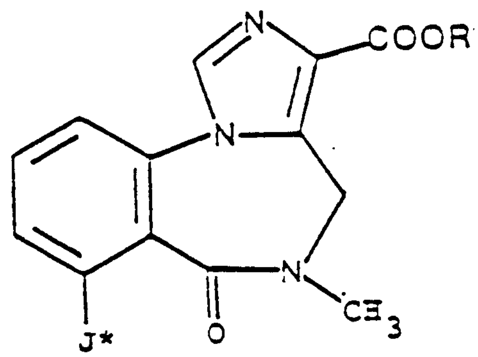

I

dadurch gekennzeichnet, dass man eine Jod$^{123}$-Lösung mit entweder

a) einer Verbindung entsprechend Formel I, jedoch mit einem stabilen nicht-radioaktiven Isotop von Jod oder

b) einer Verbindung der Formel

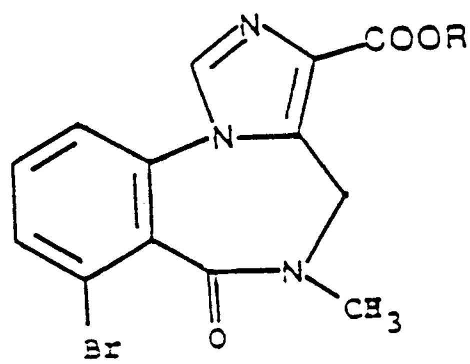

II

erhitzt.

**2.** Verfahren nach Anspruch 1, dadurch gekennzeichnet, dass Jod$^{123}$ als radioaktives Isotop von Jod verwendet wird.

**3.** Verfahren nach Anspruch 2, dadurch gekennzeichnet, dass man Aethyl-7-$^{123}$-jod-5,6-dihydro-5-methyl-6-oxo-imidazo-[1,5-a][1,4]-benzodiazepin-3-carboxylat herstellt.

**Claims**

**Claims for the following Contracting States : AT, BE, CH, DE, FR, GB, IT, LI, LU, NL, SE**

**1.** A radioiodinated benzodiazepine derivative of the formula

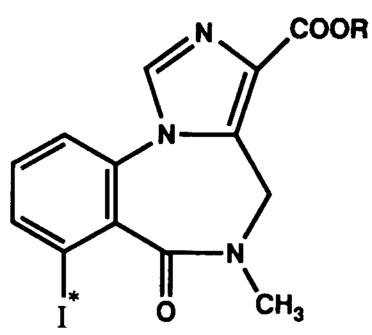

I

wherein R represents lower alkyl with 1-4 C-atoms and I* represents a radioactive iodine.

**2.** A compound in accordance with claim 1, characterized in that the radioactive iodine is iodine 123.

**3.** Ethyl 7-$^{123}$iodo-5,6-dihydro-5-methyl-6-oxo-4H-imidazo[1,5-a][1,4]benzodiazepine-3-carboxylate.

**4.** A process for the preparation of a compound of formula I of claim 1, characterized by either
a) labeling a compound corresponding to formula I, but having a stable non-radioactive isotope of iodine, with a radioactive iodine, or
b) reacting a compound of the formula

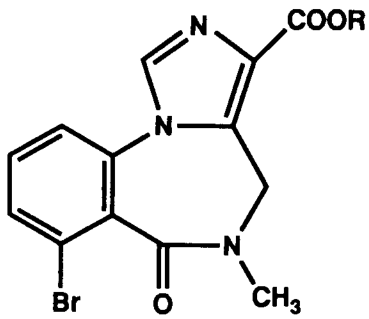

II

with a radioactive iodine.

**5.** A process according to claim 4, characterized in that iodine 123 is used as the radioactive isotope of iodine.

**6.** A composition for imaging the brain containing a compound of the formula

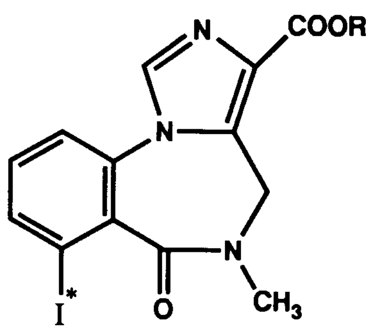

I

wherein I* signifies a radioactive iodine and R signifies an alkyl group with 1-4 C-atoms, and a suitable carrier for injection purposes.

**7.** A composition in accordance with claim 6, characterized in that the compound is ethyl 7-$^{123}$iodo-5,6-dihydro-5-methyl-6-oxo-imidazo[1,5-a][1,4]benzodiazepine-3-carboxylate.

**8.** A composition in accordance with claim 6, containing approximately 2 to approximately 10 mCi of the compound of formula I.

**Claims for the following Contracting State : ES**

**1.** A process for the preparation of a compound of the formula

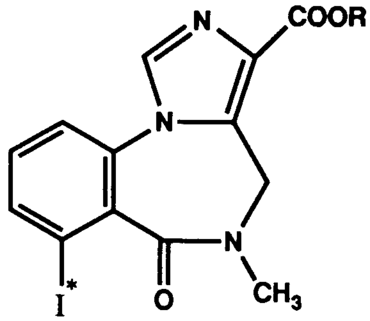

I

characterized by heating an iodine[123] solution with either

a) a compound corresponding to formula I but having a stable non-radioactive isotope of iodine, or

b) a compound of the formula

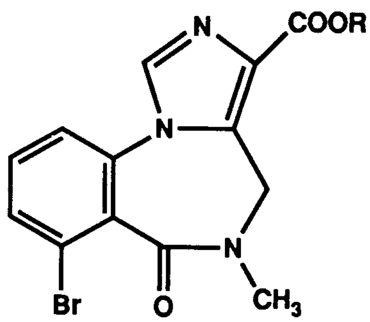

II.

**2.** A process according to claim 1, characterized in that iodine[123] is used as the radioactive isotope of iodine.

**3.** A process according to claim 2, characterized in that ethyl 7-[123]iodo-5,6-dihydro-5-methyl-6-oxo-imidazo[1,5-a][1,4]benzodiazepine-3-carboxylate is prepared.

**Revendications**

**Revendications pour les Etats contractants suivants : AT, BE, CH, DE, FR, GB, IT, LI, LU, NL, SE**

**1.** Dérivé de benzodiazépine radio-iodé de formule

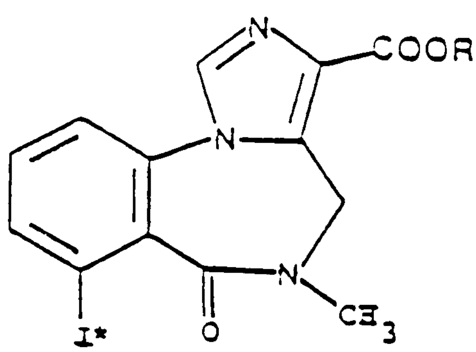

I

dans laquelle R représente un alcoyle inférieur en $C_1$ à $C_4$ et I* représente un atome d'iode radioactif.

**2.** Composé selon la revendication 1, caractérisé en ce que l'iode radioactif est l'iode 123.

**3.** 7-$^{123}$iodo-5,6-dihydro-5-méthyl-6-oxo-4H-imidazo-[1,5-a][1,4]benzodiazépine-3-carboxylate d'éthyle.

**4.** Procédé de préparation d'un composé de formule I de la revendication 1, caractérisé en ce que ou bien

a) on marque un composé correspondant à la formule I, mais avec un isotope stable non-radioactif de l'iode, avec un iode radioactif, ou bien

b) on fait réagir un composé de formule

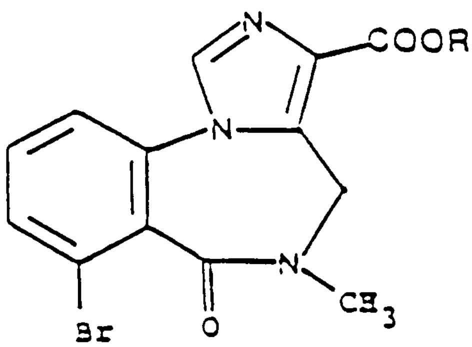

II

avec un iode radioactif.

**5.** Procédé selon la revendication 4, caractérisé en ce qu'on utilise l'iode 123 comme isotope radioactif de l'iode.

**6.** Composition pour la représentation du cerveau, contenant un composé de formule

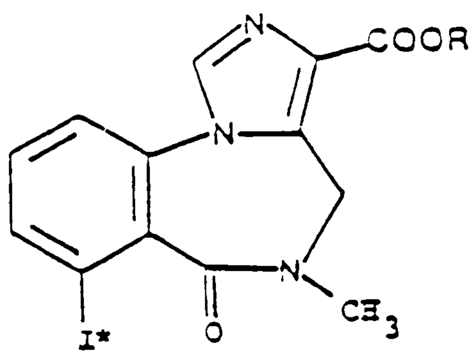

I

dans laquelle $I^*$ représente un atome d'iode radioactif et R représente un groupe alcoyle en $C_1$ à $C_4$ , et un support approprié pour des buts d'injection.

**7.** Composition selon la revendication 6, caractérisée en ce que le composé est le 7-$^{123}$-iodo-5,6-dihydro-5-méthyl-6-oxo-imidazo [1,5-a] [1,4] benzodiazépine-3-carboxylate d'éthyle.

**8.** Composition selon la revendication 6 contenant d'environ 2 à environ 10 mCi du composé de formule I.

**Revendications pour l'Etat contractant suivant : ES**

**1.** Procédé de préparation d'un composé de formule

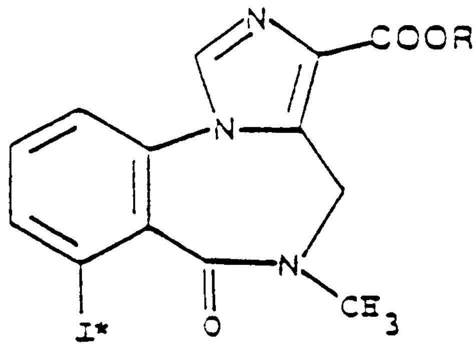

I

caractérisé en ce qu'on chauffe une solution d'iode[123] avec ou bien

a) un composé de formule I correspondante, mais avec un isotope stable non radioactif de l'iode, ou bien

b) un composé de formule

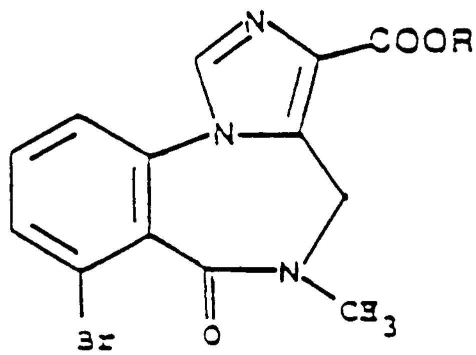

II

2. Procédé selon la revendication 1, caractérisé en ce qu on utilise l'iode[123] comme isotope radioactif de l'iode.

3. Procédé selon la revendication 2, caractérisé en ce qu'on prépare le 7-[123]-iodo-5,6-dihydro-5-méthyl-6-oxo-imidazo-[1,5-a] [1,4]-benzodiazépine-3-carboxylate d'éthyle.